Europäisches Patentamt

(19) European Patent Office

Office européen des brevets

(11) **EP 0 536 329 B1**

(12) **EUROPEAN PATENT SPECIFICATION**

(45) Date of publication and mention
of the grant of the patent:
**17.04.1996 Bulletin 1996/16**

(21) Application number: **91914562.3**

(22) Date of filing: **26.06.1991**

(51) Int. Cl.[6]: **A61K 31/505**, C07D 405/12

(86) International application number: **PCT/US91/04562**

(87) International publication number:
**WO 92/00073 (09.01.1992 Gazette 1992/02)**

(54) **R(+)-TERAZOSIN**

R(+)-TERAZOSIN

R(+)-TERAZOSINE

(84) Designated Contracting States:
**AT BE CH DE DK ES FR GB GR IT LI LU NL SE**

(30) Priority: **29.06.1990 US 546349**

(43) Date of publication of application:
**14.04.1993 Bulletin 1993/15**

(73) Proprietor: **ABBOTT LABORATORIES**
**Abbott Park, Illinois 60064-3500 (US)**

(72) Inventors:
 • **KYNCL, John, J.**
**Lake Forest, IL 60045 (US)**
 • **HORROM, Bruce, W.**
**Waukegan, IL 60087 (US)**

(74) Representative: **Modiano, Guido, Dr.-Ing.**
**Modiano & Associati S.r.l.**
**Via Meravigli, 16**
**I-20123 Milano (IT)**

(56) References cited:
**US-A- 4 026 894          US-A- 4 251 532**

 • INT.J.CLIN.PHARMACOL.THER.TOXICOL. vol. 27, no. 8, August 1989, pages 392 - 397 H.LEPOR ET AL. 'The safety and efficacy of terazosin for the treatment of benign prostatic hyperplasia'
 • AM.J.CARDIOL. vol. 65, no. 9, 1 March 1990, pages 638 - 643 R.D.MAGORIEN ET AL. 'Rest and Exercise Cardiovascular Effects of Terazosin in Congestive Heart Failure'
 • **MED.CLIN.NORTH AM. vol. 72, no. 2, March 1988, pages 441 - 448 W.H.FRISHMAN ET AL. 'Terazosin: A New Long-Acting alpha1-Adrenergic Antagonist for Hypertension'**
 • **J.HYPERTENS. vol. 6, no. SUP2, December 1988, pages S3 - S11 P.A.VAN ZWIETEN 'Basic pharmacology of alpha-adrenoceptor antagonists and hybrid drugs'**
 • **ARZNEIMITTELFORSCHUNG vol. 39, no. 1, October 1989, pages 1289 - 1291 U.DUNZENDORFER 'Effects of Terazosin in the Treatment of Benign Prostatic Hyperplasia'**
 • **PROSTATE no. SUP3, 1990, pages 75 - 84 H.LEPOR 'Role of Alpha-Adrenergic Blockers in the Treatment of Benign Prostatic Hyperplasia'**
 • **PROSTATE no. SUP3, 1990, pages 85 - 93 P.G.FABRICIUS ET AL. 'Efficacy of Once-A-Day Terazosin in Benign Prostatic Hyperplasia: A Randomized, Double-Blind Placebo-Controlled Clinical Trial'**
 • **PROSTATE vol. 20, no. 2, 1992, N.Y. pages 159 - 165 S.MERETYK ET AL. 'Alpha1-Adrenoceptor Properties of Terazosin HCI and Its Enantiomers in the Human Prostate and Canine Brain'**
 • **"Chem. Pharm. Bull", Volume 35, No. 4, 1987, T. NAGATOMO et al., "Andrenergic and Serotonergic receptor blocking potencies of Tetrazosin, a new antihypertensive agent, as assessed by Radioligand binding Assay", pages 1629-1632**

Remarks:
The file contains technical information submitted after the application was filed and not included in this specification

## Description

This invention relates to compounds having pharmacological activity and to pharmaceutical compositions containing such compounds. More particularly, this invention concerns $R$(+)-2-[4-[(tetrahydro-2-furanyl)carbonyl]-1-piperazinyl]-6,7-dimethoxy-4-quinazolinamine, substantially free of the S(-)-enantiomer, its pharmaceutically acceptable salts and hydrates and to pharmaceutical compositions containing the compound.

Background of the Invention

The adrenergic nervous control of bodily functions is mediated by two hormones: norepinephrine, which is generated in the adrenergic nerves and released from their endings, and epinephrine, which is synthesized in the adrenal medulla and secreted into circulating blood. Both of these hormones act by binding to special receptors, designated as "adrenergic" receptors, which mediate the signal of the hormones to the intracellular biochemical mechanisms leading to stimulation of diverse physiological functions. Such functions include contraction of vascular smooth muscle (which can increase blood pressure), acceleration of heart rate, induction of metabolic changes in the liver, modulation of central nervous system activity, and many others.

The adrenergic receptors are proteins embedded in celluler membranes having unique, specific amino acid sequences. Four general families of adrenergic receptors have been identified and designated $\alpha_1$, $\alpha_2$, $\beta_1$ and $\beta_2$, all of which can be stimulated by norepinephrine and epinephrine. These receptor families, however, differ such that specific agents have been developed which can selectively stimulate or inhibit each type of receptor. The degree and type of receptor selectivity for a particular agonist or antagonist agent is an important pharmacological property of such an agent and can have substantial impact on its biological activity, side effects and safety. In general, excessive stimulation of the $\alpha_1$ adrenoreceptor is a hallmark of numerous pathological situations and disease states such as hypertension, congestive heart failure, cardiac hyperplasia, benign prostatic hyperplasia, hyperinsulinemia, lipid disorders, impotency, as well as many others.

Importantly, the $\alpha_2$ adrenoreceptors, which are very similar to the $\alpha_1$ species, regulate the release of the two adrenergic hormones, norepinephrine and epinephrine, and impact on the overall level of adrenergic activity. The stimulation of $\alpha_2$ receptors by an agonist inhibits the secretion of norepinephrine and epinephrine, whereas $\alpha_2$ antagonist activity increases the secretion of these hormones substantially. Thus, the $\alpha_1/\alpha_2$ adrenoreceptor selectivity of an $\alpha$-antagonist is very important and a desirable feature.

A number of non-selective $\alpha$-adrenergic blockers, such as phenoxybenzamine and phentolamine, have prominent effects on both $\alpha_1$, and $\alpha_2$ receptors. It is the $\alpha_2$ component of their adrenergic receptor activity which increases the adrenergic hormone secretion and thus limits their therapeutic use. Typical of such $\alpha_2$ antagonist effects are increases in plasma catecholamine levels, increases in heart rate and contractility, and other highly undesirable therapeutic phenomena.

It is therefore desirable to obtain $\alpha_1$ blocking agents which have greater $\alpha_1/\alpha_2$ selectivity than agents currently available. Such selectivity permits treatment of diseases characterized by elevated $\alpha_1$ adrenergic activity without stimulating $\alpha_2$ adrenoreceptor-mediated secretion of norepinephrine and epinephrine.

2-[4-[(Tetrahydro-2-furanyl)carbonyl]-1-piperazinyl]-6,7-dimethoxy-4-quinazolinamine, also commonly known by its generic name, terazosin, has been known for several years as an antihypertensive drug. United States Patent 4,026,894 discloses and claims the compound and United States Patent 4,112,097 discloses and claims pharmaceutical compositions containing the compound and a method of treating hypertension in mammals. United States Patent 4,251,532 discloses and claims the dihydrate of the hydrochloride salt of terazosin. The latter patent also discloses and claims pharmaceutical compositions comprising the hydrochloride dihydrate and a method of treating hypertension. While the terazosin molecule possesses a single chiral center, and can thus exist in two enantiomeric forms, none of these patents discusses this optical property of the molecule or mentions the two enantiomers.

In 1987, Nagatomo and coworkers reported the binding of the racemic compound and the individual enantiomers to $\alpha$-receptors in dog brain and aorta tissue (Nagatomo, et al., Chem. Pharm. Bull., **35(4)**: 1629-1632 (1987)). Their data indicate that, while both enantiomers and the racemic compound bind selectively to the $\alpha_1$ receptors, little difference appeared to exist between the degrees of selectivity of the two enantiomers for $\alpha_1$ receptors over $\alpha_2$ receptors. This article did not report any data to indicate the optical purity of the materials employed.

Summary of the Invention

It has now been found that the two enantiomeric forms of 2-[4-[(tetrahydro-2-furanyl)carbonyl]-1-piperazinyl]-6,7-dimethoxy-4-quinazolinamine (terazosin) can be resolved, and a significant difference exists in the degree of selective binding the the R(+)-and S(-)-enantiomers at the $\alpha$-adrenergic receptors, resulting in important unexpected pharmacological properties and in their toxicity. The lack of affinity of the R(+)-enantiomer of terazosin for the $\alpha_2$ receptors compared

to that of either the S(-)-enantiomer or the racemic compound is believed to confer advantages on the R(+)-enantiomer as a pharmaceutical agent.

The present invention thus provides, in one embodiment, the product R(+)-2-[4-[(tetrahydro-2-furanyl)carbonyl]-1-piperazinyl]-6,7-dimethoxy-4-quinazolinamine, its pharmaceutically acceptable salts and hydrates, having an enantiomeric purity of at least 91%.

In another embodiment, there are provided pharmaceutical compositions comprising a therapeutically effective amount of R(+)-2-[4-[(tetrahydro-2-furanyl)carbonyl]-1-piperazinyl]-6,7-dimethoxy-4-quinazolinamine, a pharmaceutically acceptable salt and/or hydrate thereof, having an enantiomeric purity of at least 91%, in combination with a pharmaceutically acceptable carrier.

In a further embodiment of the present invention there is provided the use of R(+)-2-[4-[(tetrahydro-2-furanyl)carbonyl]-1-piperazinyl]-6,7-dimethoxy-4-quinazolinamine or a pharmaceutically acceptable salt or hydrate thereof having an enantiomeric purity of at least 91% for manufacturing a medicament for treating disease states characterized by abnormally elevated levels of $\alpha_1$ adrenergic activity, particularly hypertension, congestive heart failure, hyperinsulinemia and benign prostatic hyperplasia, in a mammal in need of such treatment.

Detailed Description

R(+)-2-[4-[(tetrahydro-2-furanyl)carbonyl]-1-piperazinyl]-6,7-dimethoxy-4-quinazolinamine or a pharmaceutically acceptable salt and/or hydrate thereof, substantially free of the S(-)-enantiomer has utility for the treatment or amelioration of disease states which are modulated by $\alpha$-adrenergic receptor blocking agents. These disease states are recognized in the literature to include hypertension, congestive heart failure, cardiac arrhythmia, pulmonary hypertension, arterioconstriction, and benign prostatic hyperplasia (see, for example, W. H. Frishman and Shlomo Charlap, "Adrenergic Receptors as Pharmacological Targets: The Alpha-Adrenergic Blocking Drugs," Chapter 4 in *Adrenergic Receptors in Man,* Paul A. Insel, Ed., Marcel Dekker, Inc., New York.

The term "pharmaceutically acceptable salts" refers to the relatively non-toxic, inorganic and organic acid addition salts of the compound of the present invention. These salts can be prepared *in situ* during the final isolation and purification of the compounds or by separately reacting the purified compound in its free base form with a suitable organic or inorganic acid and isolating the salt thus formed. Representative salts include the hydrobromide, hydrochloride, sulfate, bisulfate, phosphate, nitrate, acetate, oxalate, valerate, oleate, palmitate, stearate, laurate, borate, benzoate, lactate, phosphate, tosylate, citrate, maleate, fumarate, succinate, tartrate, naphthylate, mesylate, glucoheptonate lactobionate, laurylsulphonate salts and the like. (See, for example S. M. Berge, et al., "Pharmaceutical Salts," J. Pharm. Sci., **66**: 1-19 (1977) which is incorporated herein by reference.) The particularly preferred salt of this invention is the hydrochloride.

In accordance with the present invention, the two enantiomers have now been substantially completely resolved, and the optical rotations of the base form of the compound have been found to be $[a]_D^{22} = 34.83°$ (C=1, 3N hydrochloric acid) for the R(+)-enantiomer and $[a]_D^{22} = -26.9°$ (C=1, 3N hydrochloric acid) for the S(-)-enantiomer. Conversion of the base forms of the two enantiomers to the hydrochloride salt dihydrate forms has produced materials having optical rotations of $[a]_D^{28.5} = +23.9°$ (C=1, $H_2O$) or greater for the dextrorotatory (i.e. R(+)) enantiomer and $[a]_D^{28.5} = -23.1°$ (C=1, $H_2O$) for the levorotatory (i.e. S(-)) enantiomer. Further purification of the R(+)-enantiomer hydrochloride dihydrate has produced material having an optical rotation of $[a]_D^{24} = +25.3°$ (C=1, $H_2O$).[5]

The binding affinity of the substantially completely resolved enantiomers of terazosin for the $\alpha_1$ and $\alpha_2$ adrenoreceptors (including the $\alpha_{2A}$ and $\alpha_{2B}$ receptor subtypes) was measured using standard techniques and the results are presented below in Table 1. It is known that more than one species of $\alpha_2$ adrenoreceptor can be differentiated in different tissues (D. B. Bylund, *Pharmacol. Biochem. & Behavior,* **22**: 835-843 (1985). For example, human platelets have been recognized to contain an almost pure population of a sub-type of $\alpha_2$ adrenoreceptor designated $\alpha_{2A}$, whereas neonatal rat lung cellular membranes contain an $\alpha_2$ adrenoreceptor subtype which has been designated $\alpha_{2B}$. The cellular membranes of rat cerebral cortex have roughly equal numbers of $\alpha_2$ adrenoreceptors of both the $\alpha_{2A}$ and $\alpha_{2B}$ subtypes.

Binding of a compound at $\alpha_1$ and $\alpha_2$ receptor sites is typically determined by allowing the test compound to compete with radiolabeled compounds which are known to selectively bind at each site. The technique is well known and described in the literature. The $pK_I$, or negative logarithm of the binding equilibrium constant, is determined from the experimental data for each receptor and the degree of selectivity of the compound in question for $\alpha_1$ over $\alpha_2$ receptors can be measured by the antilogarithm of the differences between the $pK_I$ values for the two receptors.

$\alpha_1$ Adrenergic binding data were obtained for the two enantiomers of terazosin and the racemic compound. Tissue from the liver and cerebral cortex of male Sprague-Dawley rats was homogenized in ice-cold assay buffer (Tris-HCl, 50 mM, pH 7.0 and 22°C). After centrifugation at 48,000 g for ten minutes the resulting pellets, containing cellular membranes containing $\alpha$-receptors, were resuspended in 20 volumes of assay buffer and recentrifuged for ten minutes at 48,000 g. Liver membranes were diluted 200-fold and cerebral cortex tissues 50-fold with assay buffer.

Binding to $\alpha_1$ adrenergic receptors was characterized with liver membranes in competition studies using tritiated prazosin (82 Ci/mmol, DuPont NEN; 0.2 nM) and six concentrations of each test compound at half-log incremental concentrations. Binding to $\alpha_2$ adrenergic receptors was characterized in cerebral cortex tissue using tritiated rauwolscine (82.2 Ci/mMol, DuPont NEN; 0.5 nM) and six concentrations of competing test compound. Equilibrium binding was characterized after a 50 minute incubation period at 22°C. Bound radioligand was separated from radioligand in solution by filtration under vacuum through Whatman 935 AH filters. After washing five times with ice-cold assay buffer, the filters were immersed in 3 ml of Ready-Solv EP Scintillation fluid (Beckman) and counted in a Beckman LS3801 counter for 10 minutes or to a preset counting error of 4.5% at 50% counting efficiency.

To determine $\alpha_{2A}$ adrenoreceptor binding affinities, human platelets were harvested using the techniques described in the literature (Newman, et al., *J. Clin. Invest.*, **61**: 395-402 (1978); Hoffman, et al., *Proc. Nat.. Acad. Sci.* **77**: 4569-4573 (1980); and Hoffman, et al., *Endocrinology*, **110**: 926-932 (1982)) These harvested platelets were used as a source of $\alpha_{2A}$ adrenoreceptors. Neonatal rat lung tissue was used as the source of $\alpha_{2B}$ adrenoreceptors and was prepared by the techniques described by Bylund, et al., *J. Pharm. Exp. Ther.*, **245**: 600-607 (1988). Basically, in each instance, tissues were homogenized and a cell membrane fraction prepared and washed by centirfugation, with a final resuspension of the cell homogenate in 6.25 volumes of 25 mM glycylglycine buffer (pH 7.4) and stored in 2 ml aliquots at - 80° C until used in the assay. Each assay was performed as described above, using 50 $\mu$L of compound, water, or $10^{-5}$M phentolamine (to define non-specific binding), plus 450 $\mu$L of tritiated rauwolscine (approximately 0.2 nM) and 500 $\mu$L of tissue homogenate which, just prior to the assay was diluted with a further 12 volumes of gycylglycine buffer. The tube contents were mixed and allowed to equilibrate for two hours at O° C. Radioligand bound to the receptors was separated from free radioligand using rapid filtration over Whatman GF/B filters. The retained tissue was rinsed with 45 mL washes of 50mM TRIS-HCl (pH 7.4) buffer. The filters were placed in individual scintillation vials, dried, and immersed in 3 mL of scintillation fluid. Radioactivity was determined using standard scintillation counting techniques.

The concentration at which 50% of the specifically bound radioligand was displaced by the test compound ($IC_{50}$) was calculated and converted to an equilibrium dissociation constant ($K_I$) using the formula:

$$K_I = IC_{50}/(1 + [L]/K_D)$$

where [L] is the concentration of radioligand and $K_D$ is the equilibrium dissociation constant of the radioligand for the receptor. The mean $pK_I$ values for the R(+), S(-), and racemic terazosin appear in Table 1.

Table 1

| $\alpha$-Adrenergic Binding of *rac*-Terazosin and Its Enantiomers | | | | | | | |
|---|---|---|---|---|---|---|---|
| Cmpd. | $\alpha_1$ Receptor Rat Liver $pK_I$ ($\pm$SEM) | $\alpha_{2A+2B}$ Receptor Rat Cortex $pK_I$ ($\pm$SEM) | $\alpha_1/\alpha_2$ Selectivity Ratio | $\alpha_{2A}$ Receptor Human platelets $pK_I$ ($\pm$SEM) | $\alpha_1/\alpha_2$ Selectivity Ratio | $\alpha_{2B}$ Receptor Rat Lung $pK_I$ ($\pm$SEM) | $\alpha_1/\alpha_2$ Selectivity Ratio |
| R(+) | 9.016 $\pm$0.083 | 5.938$\pm$0.178 | 1,197 | 5.668$\pm$0.033 | 2,260 | 6.603$\pm$0.041 | 260 |
| Racemic | 9.912 $\pm$0.101 | 6.569$\pm$0.129 | 420 | 6.285$\pm$0.052 | 811 | 8.250$\pm$0.078 | 8.8 |
| S(-) | 9.192 $\pm$0.112 | 7.036$\pm$0.199 | 143 | 6.538$\pm$0.039 | 453 | 8.777$\pm$0.119 | 2.6 |

*Antilog [$pK_I(\alpha_1)$-$pK_I(\alpha_2)$]

Examination of the data presented in Table 1 show that while R(+)-terazosin exhibited binding affinity for the $\alpha_1$ adrenoreceptor similar to that of the S(-)-enantiomer and racemate, the R(+)-enantiomer was more selective for the $\alpha_1$ adrenorecptor as indicated by the $\alpha_1/\alpha_2$ selectivity ratios for the two enantiomers and the racemate. The degree of selectivity of the R(+)-enantiomer over the S(-)-enantiomer or the racemate is more pronounced when the $\alpha_1/\alpha_2$ selectivity ratiosvalues for the $a_{2A}$ and $a_{2B}$ adrenoreceptor subtypes are compared.

As discussed above, compounds which are active at $\alpha_2$ receptors are implicated in controlling the release of norepinephrine and related catecholamines. Thus, it is believed that *R*(+)-2-[4-[(tetrahydro-2-futanyl)carbonyl]-1-piperazinyl]-6,7-dimethoxy-4-quinazolinamine possesses useful pharmaceutical properties while being less subject to undesirable side effects which flow from $\alpha_2$-adrenergic binding activity.

The acute toxicities of the two enantiomeric forms of terazosin and the racemic compound were tested in adult male mice by intravenous administration, and the data appear in Table 2.

Table 2

| Acute Toxicity | | | |
|---|---|---|---|
| Compound | $LD_{50}$ mg/Kg | Confidence 95% Limits | Statistical Significance |
| R(+) | 306.6 | 265.8 - 445.1 | Rac vs R(+) = p <0.05 |
| Racemate | 247.9 | 230.5 - 273.0 | R(+) vs S(-) = p<0.05 |
| S(-) | 204.6 | 178.8 - 234.1 | Rac vs S(-) = p<0.05 |

The data appearing in Table 2 show that R(+)-terazosin exhibits a roughly 50% higher $LD_{50}$ (i.e. lower acute toxicity) than the corresponding S(-)-enantiomer.

Racemic terazosin and the two enentiomers were tested for their effects on blood pressure and heart rate in unanesthetized spontaneously hypertensive (SH) rats, and the results are presented in Tables 3 and 4. The blood pressure of adult, male rats of the Okamoto strain to which the test compounds were administered, was measured by means of an automatically controlled pressure cuff attached to the base of the tail of each test animal. A photocell, placed distallly to the cuff sensed the arterial pulse wave. Five interference-free signals, obtained during deflation of the cuff were obtained for each rat. Only those rats having a systolic blood pressure in excess of 175 mm Hg during the control were employed in the study.

Table 3

| Effects of Terazosin on Blood Pressure in Spontaneously Hypertensive Rats | | | | | |
|---|---|---|---|---|---|
| Hours After Dosing | Dose (mg/Kg) | Vehicle % Change from 0- Hour Value (SEM) | Racemate % Change from 0- Hour Value (SEM) | R(+) % Change from 0- Hour Value (SEM) | S(-) % Change from 0- Hour Value (SEM) |
| 1 | 0.0 | -1.32 (2.8) | | | |
| | 1.0 | | -20.52 (1.6) | -22.37 (1.7) | -25.00 (1.7) |
| | 10.0 | | -36.18 (2.9) | -28.58 (2.4) | -31.81 (3.0) |
| 5 | 0.0 | -2.77 (2.5) | | | |
| | 1.0 | | -8.82 (2.7) | -6.42 (1.3) | -27.25 (2.2) |
| | 10.0 | | -22.60 (1.9) | -18.16 (2.4) | -30.38 (2.8) |

Table 4

| Effects of Terazosin on Heart Rate in Spontaneously Hypertensive Rats | | | | | |
|---|---|---|---|---|---|
| Hours After Dosing | Dose (mg/Kg) | Vehicle % Change from 0- Hour Value (SEM) | Racemate % Change from 0- Hour Value (SEM) | R(+) % Change from 0- Hour Value (SEM) | S(-) % Change from 0- Hour Value (SEM) |
| 1 | 0.0 | -2.93 (2.9) | | | |
| | 1.0 | | 8.81 (6.7) | -0.04 (3.5) | 5.86 (3.2) |
| | 10.0 | | 8.58 (6.0) | -1.68 (3.5) | 10.92 (5.2) |
| 5 | 0.0 | -9.96 (3.7) | | | |
| | 1.0 | | -4.61 (3.8) | -6.20 (2.5) | -0.31 (4.7) |
| | 10.0 | | 2.44 (6.5) | -13.26 (3.3) | 3.94 (5.4) |

The data in Table 3 shows that (R(+)-, S(-)- and racemic terazosin all produced similar lowering of blood pressure; however, the data in Table 4 show that R(+)-terazosin produced less effect on heart rate increase than either S(-)-terazosin or the racemate.

The inhibition of $\alpha_2$ adrenoreceptors *in vivo* is known to facilitate the release of the neuronal transmitter, norepinopehrine₁ which in turn can cause an increase in cardiac contractility. In a further test, racemic terazosin and the two enantiomers were tested for their effects on the levels of plasma norepinephrine in anesthetized dogs, and the results appear in Table 5. Male beagle dogs weighing between 8.2 and 13.2 Kg were anesthetized with pentobarbital. Electrocardiogram leads were attached to the dogs and a lead 11 electrocardiogram was recorded. A Swan Ganz catheter was advanced into the pulmonary artey for measurement of pulmonary artery pressure and cardiac output. Central venous pressure was measured through the proximal port of the catheter. A dual tip micromanometer (Millar Model SPC-770 7F) was advanced into the left ventricle of the heart for measurement of left ventricular pressure. The right femoral vein was cannulated for administration of the test compounds.

Following a stabilization period of sixty minutes, vehicle (0.9% NaCl) was injected at a volume of 0.1 mg/Kg. Sixty minutes later, the lower dose (0.3 mg/kg) of the test compound was administered, followed sixty minutes later by the higher dose (3.0 mg/kg). The compounds were tested in fifteen dogs each, using a randomized schedule.

Table 5
Effect of Terazosin on
Left Ventricular Contractility (dP/dt$_{max}$)
in Anesthetized Dogs

Time (Minutes) Following Administration of An Intravenous Dose of 3 mg/Kg

| | Pre-Dose | 1.5 | 2 | 4 | 6 | 8 | 10 | 20 | 30 | 40 | 50 | 60 |
|---|---|---|---|---|---|---|---|---|---|---|---|---|
| **R(+)-Terazosin** | | | | | | | | | | | | |
| N | 14 | 14 | 13 | 13 | 13 | 13 | 13 | 13 | 13 | 13 | 13 | 13 |
| Mean | 2279.64 | 2434.21 | 2432.69 | 2333.77 | 2298.23 | 2261.00 | 2249.38 | 2150.08 | 2178.08 | 2196.69 | 2207.69 | 2221.38 |
| SEM* | 77.69 | 86.68 | 85.70 | 80.49 | 81.23 | 82.45 | 77.24 | 70.03 | 76.31 | 72.70 | 68.34 | 79.73 |
| **Racemic Terazosin** | | | | | | | | | | | | |
| N | 15 | 15 | 15 | 15 | 15 | 15 | 15 | 15 | 15 | 15 | 14 | 14 |
| Mean | 2652.40 | 3255.53 | 3215.33 | 3087.00 | 3037.53 | 2936.67 | 2912.60 | 2873.60 | 2934.33 | 3071.73 | 2955.29 | 2851.57 |
| SEM* | 172.19 | 257.69 | 233.91 | 221.17 | 229.33 | 192.19 | 195.67 | 209.06 | 227.25 | 276.66 | 250.25 | 186.38 |
| **S(-)-Terazosin** | | | | | | | | | | | | |
| N | 15 | 14 | 15 | 15 | 15 | 15 | 15 | 15 | 15 | 15 | 13 | 11 |
| Mean | 2638.80 | 3352.64 | 3271.20 | 3119.73 | 3025.60 | 2975.20 | 2917.53 | 2834.33 | 2841.07 | 2855.80 | 2788.54 | 2964.55 |
| SEM* | 152.40 | 253.25 | 222.93 | 170.95 | 167.45 | 173.82 | 166.77 | 159.67 | 155.15 | 143.85 | 107.69 | 1.26 |

*SEM = Standard error of the mean

The data in Table 5 show that there was no appreciable change in left ventricular contractility upon administration of the R(+)-enantiomer of terazosin, while administration of either the S(-)-enantiomer or the racemate produced measurable increases, even one hour after administration.

The present invention also provides pharmaceutical compositions which comprise the product mentioned above formulated together with one or more non-toxic pharmaceutically acceptable carriers. The pharmaceutical compositions may be specially formulated for oral administration in solid or liquid form, for parenteral injection, or for rectal, vaginal, or topical administration.

The pharmaceutical compositions of this invention can be administered to humans and other animals orally, rectally, parenterally , intracisternally, intravaginally, intraperitoneally, topically (as by powders, ointments, or drops), bucally, or as an oral or nasal spray. The term "parenteral" administration as used herein refers to modes of administration which include intravenous, intramuscular, intraperitoneal, intrasternal, subcutaneous and intraarticular injection and infusion.

Pharmaceutical compositions of this invention for parenteral injection comprise pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol, and the like), and suitable mixtures thereof, vegetable oils (such as olive oil), and injectable organic esters such as ethyl oleate. Proper fluidity can be maintained, for example, by the use of coating materials such as lecithin, by the maintenance of the required particle size in the case of dispersions, and by the use of surfactants.

These compositions may also contain adjuvants such as preservative, wetting agents, emulsifying agents, and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents such as sugars, sodium chloride, and the like, Prolonged absorption of the injectable pharmaceutical form may be brought about by the inclusion of agents which delay absorption such as aluminum monostearate and gelatin.

In some cases, in order to prolong the effect of the drug, it is desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Injectable depot forms are made by forming microencapsuled matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides) Depot injectable formulations are also prepared by entrapping the drug in liposomes or microemulsions which are compatible with body tissues.

The injectable formulations can be sterilized, for example, by filtration through a bacterialretaining filter, or by incorporating sterilizing agents in the form of sterile solid compositions which can be dissolved or dispersed in sterile water or other sterile injectable medium just prior to use.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

The active compounds can also be in micro-encapsulated form, if appropriate, with one or more of the above-mentioned excipients.

Liquid dosage forms for oral administration include pharmaceutically acceptable emulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, and perfuming agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar, and tragacanth, and mixtures thereof.

Compositions for rectal or vaginal administration are preferably suppositories which can be prepared by mixing the compounds of this invention with suitable non-irritating excipients or carriers such as cocoa butter, polyethylene glycol or a suppository wax which are solid at room temperature but liquid at body temperature and therefore melt in the rectum or vaginal cavity and release the active compound.

Compounds of the present invention can also be administered in the form of liposomes. As is known in the art, liposomes are generally derived from phospholipids or other lipid substances. Liposomes are formed by mono- or multi-lamellar hydrated liquid crystals that are dispersed in an aqueous medium. Any non-toxic, physiologically acceptable and metabolizable lipid capable of forming liposomes can be used. The present compositions in liposome form can contain, in addition to a compound of the present invention, stabilizers, preservatives, excipients, and the like. The preferred lipids are the phospholipids and the phosphatidyl cholines (lecithins), both natural and synthetic.

Methods to form liposomes are known in the art. See, for example, Prescott, Ed., Methods in Cell Biology, Volume XIV, Academic Press, New York, N.Y. (1976), p. 33 et seq.

Dosage forms for topical administration of a compound of this invention include powders, sprays, ointments and inhalants. The active compound is mixed under sterile conditions with a pharmaceutically acceptable carrier and any needed preservatives, buffers, or propellants which may be required. Opthalmic formulations, eye ointments, powders and solutions are also contemplated as being within the scope of this invention.

Actual dosage levels of active ingredients in the pharmaceutical compositions of this invention may be varied so as to obtain an amount of the active compound(s) that is effective to achieve the desired therapeutic response for a particular patient, compositions, and mode of administration. The selected dosage level will depend upon the activity of the particular compound, the route of administration, the severity of the condition being treated, and the condition and prior medical history of the patient being treated. However, it is within the skill of the art to start doses of the compound at levels lower than required for to achieve the desired therapeutic effect and to gradually increase the dosage until the desired effect is achieved.

For use as an antihypertensive agent, the product of this invention is generally dosed orally at levels of about 0.01 mg to about 250 mg, more preferably of about 0.1 mg to about 100 mg of active compound per kilogram of body weight per day to a mammalian patient. If desired, the effective daily dose may be divided into multiple doses for purposes of administration, e.g. two to four separate doses per day.

**Example 1**

Preparation of R(+)-2-[4-[(tetrahydro-2-furanyl)carbonyl]-1-piperazinyl]-6,7-dimethoxy-4-quinazolinamine from R(+)-tetrahydro-2-furoic acid resolved as the brucine salt

Step 1 - Preparation of R(+)-tetrahydro-2-furoic acid

Using the procedure detailed in Can. J. Chem., **61**:1383-1386 (1983), racemic tetrahydro-2-furoic acid was first converted to a mixture of the diastereomeric brucine salts by reaction with (-)-brucine in ethyl acetate. The crude brucine salt of R(+)-tetrahydro-2-furoic acid which first precipitated had a melting point of 191-197°C and an optical rotation $[a]_D^{23}$ = - 7.86° (C=1, methanol). The material was recrystallized three times from ethyl acetate to yield material melting at 200-203°C and having an optical rotation $[a]_D^{23°C}$ = -4.8° (C=1, methanol) (literature value $[a]_D$ = -5.8° (C=1, methanol)).

The salt was acidified to recover R(+)-tetrahydro-2-furoic acid, b.p. 57-58°C at 0.1 mm Hg, refractive index, $\eta_D^{25}$ = 1.4953, optical rotation $[a]_D^{22°C}$ = + 33.37° (C=1, chloroform) (literature value $[a]_D$ = + 30.4° (C=1, chloroform).

Step 2 - Preparation of R(+)-2-[4-[(tetrahydro-2-furanyl)carbonyl]-1-piperazinyl]-6,7-dimethoxy-4-quinazolinamine

R(+)-Tetrahydro-2-furoic acid was dissolved in tetrahydrofuran and 2.0 g (0.017 mole) of dicyclohexylcarbodiimide was added followed by 3.50 g (0.017 mole) N-hydroxysuccinimide. The mixture was stirred overnight at room temperature. The precipitated dicyclohexylurea which formed was collected by filtration and the residue washed with a small amount of tetrahydrofuran. The solid was discarded and the washings added to the filtrate.

To the filtrate were added a solution of 4.91 g (0.017 mole) of 4-amino-6,7-dimethoxy-2-piperazinyl-4-quinazoline in tetrahydrofuran. The resulting mixture was stirred overnight at room temperature. The solid which had precipitated was collected by filtration and washed several times with tetrahydrofuran. The washings were combined with the filtrate which was evaporated to dryness. The residual solid was taken up in a 5/1 mixture of methylene chloride/methanol and the resulting mixture distilled to remove the methylene chloride. The removed methylene chloride was replaced by an equal volume of methanol, at which point the product began to crystallize from solution. The solution was allowed to cool to room temperature and stand for several hours, yielding $R(+)$-2-[4-[(tetrahydro-2-furanyl)carbonyl]-1-piperazinyl]-6,7-dimethoxy-4-quinazolinamine, m.p. 272-274°C, optical rotation $[a]_D^{22°C}$ = 34.83° (C=1, 3N hydrochloric acid).

**Example 2**

Preparation of $R(+)$-2-[4-[(tetrahydro-2-furanyl)carbonyl]-1-piperazinyl]-6,7-dimethoxy-4-quinazolinamine, hydrochloride salt dihydrate

R(+)-Terazosin hydrochloride salt dihydrate was prepared by heating an ethanol solution of $R(+)$-2-[4-[(tetrahydro-2-furanyl)carbonyl]-1-piperazinyl]-6,7-dimethoxy-4-quinazolinamine to near reflux and adding slightly more than one equivalent of concentrated aqueous hydrochloric acid. Solution occurred immediately, and the mixture was allowed to cool to room temperature and stand for several hours. The precipitate which formed was collected by filtration, washed with ethanol, and dried to yield R(+)-2-[4-[(tetrahydro-2-furanyl)carbonyl]-1-piperazinyl]-6,7-dimethoxy-4-quinazolinamine, hydrochloride salt dihydrate having a melting point of 260.5-263.5°C and an optical rotation $[a]_D^{28.5°C}$ = +23.94° (C=1, water).

**Example 3**

Preparation of R(+)-2-[4-[(tetrahydro-2-furanyl)carbonyl]-1-piperazinyl]-6,7-dimethoxy-4-quinazolinamine hydrochloride dihydrate from enzymatically resolved tetrahydro-2-furoic acid

Step 1 - Preparation of the benzyl ester of racemic tetrahydrofuroic acid

(R,S)-Tetrahydro-2-furoic acid (1.152 kg, 10.1 mol) was dissolved in 5 liters of dichloromethane. Benzyl alcohol (1.08 kg, 10 mol) was added, followed by 10 ml of concentrated sulfuric acid. The resulting mixture was heated to reflux and maintained at that temperature, with azeotropic distillation of water derived from the reaction. When the calculated amount of water had been collected, the reaction mixture was cooled to room temperature and washed with 1 liter of 5% aqueous sodium bicarbonate solution and twice with 1 liter portions of water. The solution was then dried over anhydrous magnesium sulfate, filtered, and concentrated under vacuum to yield 1.9 kg of tetrahydro-2-furoic acid benzyl ester which was found to be 92% pure by chromatographic analysis.

Step 2 - Enzymatic resolution of tetrahydro-2-furoic acid

Tetrahydro-2-furoic acid benzyl ester (412 g, 2.0 mol) was mixed with 5 liters of 0.1 M phosphate buffer and the pH adjusted to 7.0 with sodium hydroxide solution. Prozyme® 6 enzyme (10 g, Amano International Enzyme Co., Inc., P.O. Box 1000, Troy, VA 22974, Lot No. PR002511P) was added to the solution in one portion. The pH of the resulting mixture was maintained at pH 6.84-7.03 by means of a pH Stat adding 2M sodium hydroxide solution. The mixture was allowed to react under these conditions overnight.

Chloroform (500 ml) was added to quench the reaction and the mixture was stirred for an additional fifteen minutes, after which it was filtered through diatomaceous earth to remove insoluble material. The aqueous phase was extracted twice with 500 ml portions of chloroform, and the chloroform solutions were combined, dried, and evaporated. The residue was taken up in 2 liters of diethyl ether and washed twice with 500 ml portions of water, twice with 5% aqueous sodium bicarbonate, and twice with water. The ether solution was then dried over anhydrous magnesium sulfate, filtered, and the solvent evaporated to yield a water-white oil.

This material was distilled under reduced pressure to yield 1.45 g of (S)-tetrahydro-2-furoic acid, b.p. 102-105°C at 0.25 mm Hg. Hydrogenolysis of this material under standard conditions followed by distillation under reduced pressure afforded 72.73 g of (S)-tetrahydro-2-furoic acid in two fractions:

Fraction 1:     35.93 g; b.p. 72°C at 0.25 mm Hg; $\eta_D^{25}$ = 1.4595; $[a]_D^{25}$ = -33.87°
(C = 1, CHCl$_3$);

Fraction 2:     36.8 g; 35.93 g; b.p. 72°C at 0.25 mm Hg; $\eta_D^{25}$ = 1.4595; $[a]_D^{25}$ = - 33.07°
(C = 1, CHCl$_3$).

The original aqueous phase from the enzymatic reaction was concentrated to dryness under reduced pressure to yield a yellow/brown solid. This residue was taken up in 500 ml of water and 100 g of potassium acid phosphate were added. The resulting mixture was cooled over ice for thirty minutes after which 85% phosphoric acid was added to a pH of 2.0. The aqueous phase was extracted with three 500-ml portions of diethyl ether and the combined ether extracts were dried over anhydrous magnesium sulfate and evaporated to dryness to yield a water-white oil. Reduced pressure distillation of this oil yielded 155 g of predominantly (R)-tetrahydro-2-furoic acid, contaminated with the (S)-enantiomer (b.p. 65.0°C at 0.23 mm Hg.

This material was reesterified with benzyl alcohol using the method described above. Analysis of this ester by HPLC on a chiral column indicated that the ester was about 85% the (R)-enantiomer, and about 15% of the (S)-enantiomer. This mixture of benzyl esters was resubjected to enzymatic resolution using the method detailed above. Work-up of the product of this enzymatic process in the manner described above yielded, after vacuum distillation, 47.8 g of (R)-tetrahydro-2-furoic acid in two fractions:

Fraction 1: B.p. 73-77°C at 0.35 mm Hg; $\eta_D^{25}$ = 1.4595; $[a]_D^{25}$ = +32.85°
(C = 1, CHCl$_3$);

Fraction 2: 36.8 g; 35.93 g; b.p. 77-78°C at 0.4 mm Hg; $\eta_D^{25}$ = 1.4595; $[a]_D^{25}$ = +33.39°
(C = 1, CHCl$_3$).

Step 3 - Preparation of *R*(+)-2-[4-[(tetrahydro-2-furanyl)carbonyl]-1-piperazinyl]-6,7-dimethoxy-4-quinazolinamine, hydrochloride dihydrate

The title compound was prepared from the enzymatically-resolved (R)-tetrahydro-2-furoic acid using the methods of Step 3 of Example 1 and Example 2. $[a]_D^{24}$ = + 25.33° (C = 1, H$_2$O).

**Example 4** Preparation of S(-)-2-[4-[(tetrahydro-2-furanyl)carbonyl]-1-piperazinyl]-6,7-dimethoxy-4-quinazolinamine

Step 1 - Preparation of S(-)-tetrahydrofuroic acid

Using the procedure detailed in Can. J. Chem., **61**:1383-1386 (1983), racemic tetrahydro-2-furoic acid was first converted to a mixture of the diastereomeric ephedrine salts by reaction with (+)-ephedrine in ethyl acetate. The crude S(-)-ephedrine salt which first precipitated had a melting point of 114-115°C. The material was recrystallized four times from ethyl acetate to yield material melting at 115-117°C and having an optical rotation $[a]_D^{26.5°C}$ = +13.4° (C=1, methanol) (literature $[a]_D$ - +13.8°).

The salt was acidified to recover the *S*(-)-tetrahydro-2-furoic acid, b.p. 60°C at 0.5 mm Hg, refractive index, $h_D^{25}$ = 1.4582, optical rotation $[a]_D^{22}$ = -32.02° (C=1, chloroform) (literature $[a]_D$ = -30.1° (C=1, chloroform)).

Step 2 - Preparation of S(-)-2-[4-[(tetrahydro-2-furanyl)carbonyl]-1-piperazinyl]-6,7-dimethoxy-4-quinazolinamine

The procedure employed was the same as that described above in Example 1 for the *R*(+)-enantiomer, yielding S(-)-2-[4-[(tetrahydro-2-furanyl)carbonyl]-1-piperazinyl]-6,7-dimethoxy-4-quinazolinamine, m.p. 269.5-271.1°C, optical rotation $[a]_D^{22°C}$ = -26.9° (C=1, 3N hydrochloric acid).

**Example 5**

Preparation of S(-)-2-[4-[(tetrahydro-2-furanyl)carbonyl]-1-piperazinyl]-6,7-dimethoxy-4-quinazolinamine, hydrochloride salt dihydrate

The procedure employed was the same as in Example 2 for the preparation of the hydrochloride salt of the R(+)-enantiomer. M.p. 271.5-273°C (dec.), optical rotation $[a]_D^{28.5°C}$ = -23.1° (C=1, water).

**Example 6**

Determination of the Optical Purity of R(+)-Terazosin

The material of Examples 2 and 3 was analyzed for optical purity by separation of the R(+)-and S(-)-enantiomers on a chiral AGP column ($\alpha_1$ acid glycoprotein column, ChromTech, Box 512, S-145 63 Norsberg, Sweden). The mobile phase consisted of 50 mM potassium phosphate at pH 7.4 and acetonitrile in a ratio of 94/6. The flow rate was 0.9 ml/min. The mobile phase was equilibrated at 0°C-6°C. Detection of the eluate was by ultraviolet light at 254 nM. Samples of 5µl containing 0.1 mg/ml of compound were employed.

| Example | Optical Rotation | Percent R(+)-Enantiomer |
|---|---|---|
| 2 | + 23.94° | 91 |
| 3 | + 25.33° | >99 |

## Claims

**Claims for the following Contracting States : AT, BE, CH, LI, DE, DK, FR, GB, IT, LU, NL, SE**

1. The product consisting of R(+)-2-[4-[(tetrahydro-2-furanyl)carbonyl]-1-piperazinyl]-6,7-dimethoxy-4-quinazolinamine having an enantiomeric purity of at least 91% or a pharmaceutically acceptable salt or hydrate thereof having the same enantiomeric purity.

2. The product consisting of R(+)-2-[4-[(tetrahydro-2-furanyl)carbonyl]-1-piperazinyl]-6,7-dimethoxy-4-quinazolinamine hydrochloride dihydrate having an enantiomeric purity of at least 91%.

3. A pharmaceutical composition comprising a therapeutically acceptable amount of the product as defined by Claim 1 or 2 in combination with a pharmaceutically acceptable carrier.

4. A product as defined in Claim 1 or 2 for use as a therapeutic agent.

5. Use of a product as defined by Claim 1 or 2 for manufacturing a medicament for treating disease states characterized by abnormally elevated levels of $\alpha_1$ adrenergic activity in a mammal in need of such treatment.

6. Use of a product as defined by Claim 1 or 2 for manufacturing a medicament for treating hypertension in a mammal in need of such treatment.

7. Use of a product as defined by Claim 1 or 2 for manufacturing a medicament for treating benign prostatic hyperplasia in a mammal in need of such treatment.

8. Use of a product as defined by Claim 1 or 2 for manufacturing a medicament for treating hyperinsulinemia in a mammal in need of such treatment.

9. Use of a product as defined by Claim 1 or 2 for manufacturing a medicament for treating congestive heart failure in a mammal in need of such treatment.

**Claims for the following Contracting State : ES**

1. A process for preparing a product consisting of R(+)-2-[4-[(tetrahydro-2-furanyl)carbonyl]-1-piperazinyl]-6,7-dimethoxy-4-quinazolinamine having an enantiomeric purity of at least 91% or a pharmaceutically acceptable salt or hydrate thereof having the same enantiomeric purity comprising the steps of

    a) resolving racemic tetrahydro-2-furoic acid to obtain R(+)-tetrahydro-2-furoic acid;
    b) reacting the product of step a) with 4-amino-6,7-dimethoxy-2-piperazinyl-quinazoline in a polar, aprotic organic solvent to produce R(+)-2-[4-[(tetrahydro-2-furanyl)carbonyl]-1-piperazinyl]-6,7-dimethoxy-4-quinazolinamine; and
    c) thereafter converting the product of step b) to a pharmaceutically acceptable salt or hydrate.

2. The process of Claim 1 wherein said step a) of resolving racemic tetrahydro-2-furoic acid comprises forming a mixture of diastereomeric salts by reaction of racemic tetrahydro-2-furoic acid with (-)-brucine, followed by separation of the (-)-brucine salt of R(+)-tetrahydro-2-furoic acid by recrystallization and, finally, acidification of said salt to recover R(+)-tetrahydro-2-furoic acid.

3. The process of Claim 1 wherein said step a) of resolving racemic tetrahydro-2-furoic acid comprises (1) forming the benzyl ester of racemic tetrahydro-2-furoic acid; thereafter (2) subjecting the benzyl ester of racemic tetrahydro-2-furoic acid to enzymatic hydrolysis by an enzyme which preferentially hydrolyzes the benzyl ester of S(-)-tetrahydro-2-furoic acid to form a mixture of S(-)-tetrahydro-2-furoic acid and the benzyl ester of R(+)-tetrahydro-2-furoic acid; (3) extracting the S(-)-tetrahydro-2-furoic acid from said mixture; and (4) subjecting the benzyl ester of R(+)-tetrahydro-2-furoic acid to acidic hydrolysis to recover R(+)-tetrahydro-2-furoic acid.

4. The process of Claim 1 wherein in step c) the product of step b) is converted to the hydrochloride salt dihydrate.

**Claims for the following Contracting State : GR**

1. A process for preparing a product consisting of R(+)-2-[4-[(tetrahydro-2-furanyl)carbonyl]-1-piperazinyl]-6,7-dimethoxy-4-quinazolinamine having an enantiomeric purity of at least 91% or a pharmaceutically acceptable salt or hydrate thereof having the same enantiomeric purity comprising the steps of

    a) resolving racemic tetrahydro-2-furoic acid to obtain R(+)-tetrahydro-2-furoic acid;
    b) reacting the product of step a) with 4-amino-6,7-dimethoxy-2-piperazinyl-quinazoline in a polar, aprotic organic solvent to produce R(+)-2-[4-[(tetrahydro-2-furanyl)carbonyl]-1-piperazinyl]-6,7-dimethoxy-4-quinazolinamine; and
    c) thereafter converting the product of step b) to a pharmaceutically acceptable salt or hydrate.

2. The process of Claim 1 wherein said step a) of resolving racemic tetrahydro-2-furoic acid comprises forming a mixture of diastereomeric salts by reaction of racemic tetrahydro-2-furoic acid with (-)-brucine, followed by separation of the (-)-brucine salt of R(+)-tetrahydro-2-furoic acid by recrystallization and, finally, acidification of said salt to recover R(+)-tetrahydro-2-furoic acid.

3. The process of Claim 1 wherein said step a) of resolving racemic tetrahydro-2-furoic acid comprises (1) forming the benzyl ester of racemic tetrahydro-2-furoic acid; thereafter (2) subjecting the benzyl ester of racemic tetrahydro-2-furoic acid to enzymatic hydrolysis by an enzyme which preferentially hydrolyzes the benzyl ester of S(-)-tetrahydro-2-furoic acid to form a mixture of S(-)-tetrahydro-2-furoic acid and the benzyl ester of R(+)-tetrahydro-2-furoic acid; (3) extracting the S(-)-tetrahydro-2-furoic acid from said mixture; and (4) subjecting the benzyl ester of R(+)-tetrahydro-2-furoic acid to acidic hydrolysis to recover R(+)-tetrahydro-2-furoic acid.

4. The process of Claim 1 wherein in step c) the product of step b) is converted to the hydrochloride salt dihydrate.

5. Use of a product consisting of R(+)-2-[4-[(tetrahydro-2-furanyl)carbonyl]-1-piperazinyl]-6,7-dimethoxy-4-quinazolinamine having an enantiomeric purity of at least 91% or a pharmaceutically acceptable salt or hydrate thereof having the same enantiomeric purity for manufacturing a medicament for treating disease states characterized by abnormally elevated levels of $\alpha_1$ adrenergic activity in a mammal in need of such treatment.

6. Use of a product consisting of R(+)-2-[4-[(tetrahydro-2-furanyl)carbonyl]-1-piperazinyl]-6,7-dimethoxy-4-quinazolinamine having an enantiomeric purity of at least 91% or a pharmaceutically acceptable salt or hydrate thereof having the same enantiomeric purity for manufacturing a medicament for treating hypertension in a mammal in need of such treatment.

7. Use of a product consisting of R(+)-2-[4-[(tetrahydro-2-furanyl)carbonyl]-1-piperazinyl]-6,7-dimethoxy-4-quinazolinamine having an enantiomeric purity of at least 91% or a pharmaceutically acceptable salt or hydrate thereof having the same enantiomeric purity for manufacturing a medicament for treating benign prostatic hyperplasia in a mammal in need of such treatment.

8. Use of a product consisting of R(+)-2-[4-[(tetrahydro-2-furanyl)carbonyl]-1-piperazinyl]-6,7-dimethoxy-4-quinazolinamine having an enantiomeric purity of at least 91% or a pharmaceutically acceptable salt or hydrate thereof having the same enantiomeric purity for manufacturing a medicament for treating hyperinsulinemia in a mammal in need of such treatment.

9. Use of a product consisting of R(+)-2-[4-[(tetrahydro-2-furanyl)carbonyl]-1-piperazinyl]-6,7-dimethoxy-4-quinazolinamine having an enantiomeric purity of at least 91% or a pharmaceutically acceptable salt or hydrate thereof having the same enantiomeric purity for manufacturing a medicament for treating congestive heart failure in a mammal in need of such treatment.

10. Use according to any one of Claims 5-8 wherein said product consists of R(+)-2-[4-[(tetrahydro-2-furanyl)carbonyl]-1-piperazinyl]-6,7-dimethoxy-4-quinazolinamine hydrochloride dihydrate having an enantiomeric purity of at least 91%.

## Patentansprüche

### Patentansprüche für folgende Vertragsstaaten : AT, BE, CH, LI, DE, DK, FR, GB, IT, LU, NL, SE

1. Produkt, das aus R(+)2-[4-[(Tetrahydro-2-furyl)carbonyl]-1-piperazinyl]-6,7-dimethoxy-4-chinazolinamin besteht und eine enantiomere Reinheit von wenigstens 91% aufweist, oder ein pharmazeutisch verträgliches Salz oder Hydrat davon, das die gleiche enantiomere Reinheit aufweist.

2. Produkt, das aus R(+)-2-[4-[(Tetrahydro-2-furyl)carbonyl]-1-piperazinyl]-6,7-dimethoxy-4-chinazolinaminhydrochloriddihydrat besteht, und eine enantiomere Reinheit von wenigstens 91% aufweist.

3. Pharmazeutische Zusammensetzung, die eine therapeutisch verträgliche Menge des Produktes nach Anspruch 1 oder 2 in Kombination mit einem pharmazeutisch verträglichen Träger umfaßt.

4. Produkt nach Anspruch 1 oder 2 zur Verwendung als therapeutisches Agens.

5. Verwendung eines Produktes nach Anspruch 1 oder 2 zur Herstellung eines Medikamentes zur Behandlung von Krankheitszuständen, die durch abnormal erhöhte Pegel an $\alpha_1$-adrenerger Aktivität gekennzeichnet sind, bei einem Säugerpatienten, der einer solchen Behandlung bedarf.

6. Verwendung eines Produktes nach Anspruch 1 oder 2 zur Herstellung eines Medikamentes zur Behandlung von Hochdruck bei einem Säugerpatienten, der einer solchen Behandlung bedarf.

7. Verwendung eines Produktes nach Anspruch 1 oder 2 zur Herstellung eines Medikamentes zur Behandlung von gutartiger prostatischer Hyperplasie bei einem Säugerpatienten, der einer solchen Behandlung bedarf.

8. Verwendung eines Produktes nach Anspruch 1 oder 2 zur Herstellung eines Medikamentes zur Behandlung von Hyperinsulinämie bei einem Säugerpatienten, der einer solchen Behandlung bedarf.

9. Verwendung eines Produktes nach Anspruch 1 oder 2 zur Herstellung eines Medikamentes zur Behandlung von kongestivem Herzversagen bei bei einem Säugerpatienten, der einer solchen Behandlung bedarf.

### Patentansprüche für folgenden Vertragsstaat : ES

1. Verfahren zur Herstellung eines Produktes, das aus R(+)-2-[4-[(Tetrahydro-2-furyl)carbonyl]-1-piperazinyl]-6,7-dimethoxy-4-chinazolinamin besteht und eine enantiomere Reinheit von wenigstens 91% aufweist, oder eines pharmazeutisch verträglichen Salzes oder Hydrates davon, das die gleiche enantiomere Reinheit aufweist, wobei das Verfahren folgende Schritte umfaßt:

   a) Spaltung von racemischer Tetrahydro-2-furancarbonsäure unter Erhalt von R(+)-Tetrahydro-2-furancarbonsäure;
   b) Umsetzung des Produktes nach Schritt a) mit 4-Amino-6,7-dimethoxy-2-piperazinyl-chinazolin in einem polaren aprotischen organischen Lösungsmittel unter Erhalt von R(+)-2-[4-[(Tetrahydro-2-furyl)carbonyl]-1-piperazinyl]-6,7-dimethoxy-4-chinazolinamin, und
   c) die anschließende Überführung des Produktes nach Schritt b) in ein pharmazeutisch verträgliches Salz oder Hydrat.

2. Verfahren nach Anspruch 1, wobei der Schritt a) der Spaltung der racemischen Tetrahydro-2-furancarbonsäure die Ausbildung einer Mischung von diastereomeren Salzen mittels Reaktion der racemischen Tetrahydro-2-furancarbonsäure mit (-)-Brucin umfaßt, gefolgt von der Abtrennung des (-)-Brucinsalzes von R(+)-Tetrahydro-2-furancarbonsäure durch Umkristallisation und zuletzt durch Ansäuern des Salzes, um R(+)-Tetrahydro-2-furancarbonsäure zu erhalten.

3. Verfahren nach Anspruch 1, wobei der Schritt a) der Spaltung der racemischen Tetrahydro-2-furancarbonsäure folgendes umfaßt: (1) die Ausbildung des Benzylesters von racemischer Tetrahydro-2-furancarbonsäure, danach (2) die Unterwerfung des Benzylesters von racemischer Tetrahydro-2-furancarbonsäure einer enzymatischen Hydrolyse durch ein Enzym, das vorzugsweise den Benzylester von S(-)-Tetrahydro-2-furancarbonsäure hydrolysiert, unter Ausbildung einer Mischung aus S(-)-Tetrahydro-2-furancarbonsäure und dem Benzylester von R(+)-Tetrahydro-2-furancarbonsäure, (3) die Extraktion der S(-)-Tetrahydro-2-furancarbonsäure aus der Mischung, und (4) das Unterwerfen des Benzylesters von R(+)-Tetrahydro-2-furancarbonsäure einer sauren Hydrolyse, um R(+)-Tetrahydro-2-furancarbonsäure zu erhalten.

4. Verfahren nach Anspruch 1, wobei in Schritt c) das Produkt nach Schritt b) in das Hydrochloridsaizdihydrat überführt wird.

**Patentansprüche für folgenden Vertragsstaat : GR**

1. Verfahren zur Herstellung eines Produktes, das aus R(+)-2-[4-[(Tetrahydro-2-furyl)carbonyl]-1-piperazinyl]-6,7-dimethoxy-4-chinazolinamin besteht und eine enantiomere Reinheit von wenigstens 91% aufweist, oder eines pharmazeutisch verträglichen Salzes oder Hydrates davon, das die gleiche enantiomere Reinheit aufweist, wobei das Verfahren folgende Schritte umfaßt:

   a) Spaltung von racemischer Tetrahydro-2-furancarbonsäure unter Erhalt von R(+)-Tetrahydro-2-furancarbonsäure;
   b) Umsetzung des Produktes nach Schritt a) mit 4-Amino-6,7-dimethoxy-2-piperazinyl-chinazolin in einem polaren aprotischen organischen Lösungsmittel unter Erhalt von R(+)-2-[4-[(Tetrahydro-2-furyl)carbonyl]-1-piperazinyl]-6,7-dimethoxy-4-chinazolinamin, und
   c) die anschließende Überführung des Produktes nach Schritt b) in ein pharmazeutisch verträgliches Salz oder Hydrat.

2. Verfahren nach Anspruch 1, wobei der Schritt a) der Spaltung der racemischen Tetrahydro-2-furancarbonsäure die Ausbildung einer Mischung von diastereomeren Salzen mittels Reaktion der racemischen Tetrahydro-2-furancarbonsäure mit (-)-Brucin umfaßt, gefolgt von der Abtrennung des (-)-Brucinsalzes von R(+)-Tetrahydro-2-furancarbonsäure durch Umkristallisation und zuletzt durch Ansäuern des Salzes, um R(+)-Tetrahydro-2-furancarbonsäure zu erhalten.

3. Verfahren nach Anspruch 1, wobei der Schritt a) der Spaltung der racemischen Tetrahydro-2-furancarbonsäure folgendes umfaßt: (1) die Ausbildung des Benzylesters von racemischer Tetrahydro-2-furancarbonsäure, danach (2) die Unterwerfung des Benzylesters von racemischer Tetrahydro-2-furancarbonsäure einer enzymatischen Hydrolyse durch ein Enzym, das vorzugsweise den Benzylester von S(-)-Tetrahydro-2-furancarbonsäure hydrolysiert, unter Ausbildung einer Mischung aus S(-)-Tetrahydro-2-furancarbonsäure und dem Benzylester von R(+)-Tetrahydro-2-furancarbonsäure, (3) die Extraktion der S(-)-Tetrahydro-2-furancarbonsäure aus der Mischung, und (4) das Unterwerfen des Benzylesters von R(+)-Tetrahydro-2-furancarbonsäure einer sauren Hydrolyse, um R(+)-Tetrahydro-2-furancarbonsäure zu erhalten.

4. Verfahren nach Anspruch 1, wobei in Schritt c) das Produkt nach Schritt b) in das Hydrochloridsalzdihydrat überführt wird.

5. Verwendung eines Produktes, das aus R(+)-2-[4-[(Tetrahydro-2-furyl)carbonyl]-1-piperazinyl]-6,7-dimethoxy-4-chinazolinamin besteht und eine enantiomere Reinheit von wenigstens 91% aufweist, oder eines pharmazeutisch verträglichen Salzes oder Hydrates davon, das die gleiche enantiomere Reinheit aufweist, zur Herstellung eines Medikamentes zur Behandlung von Krankheitszuständen, die durch abnormale erhöhte Pegel an $\alpha_1$-adrenerger Aktivität gekennzeichnet sind, bei einem Säugerpatienten, der einer solchen Behandlung bedarf.

6. Verwendung eines Produktes, das aus R(+)-2-[4-[(Tetrahydro-2-furyl)carbonyl]-1-piperazinyl]-6,7-dimethoxy-4-chinazolinamin besteht und eine enantiomere Reinheit von wenigstens 91% aufweist, oder eines pharmazeutisch verträglichen Salzes oder Hydrates davon, das die gleiche enantiomere Reinheit aufweist, zur Herstellung eines Medikamentes zur Behandlung von Hochdruck bei einem Säugerpatienten, der einer solchen Behandlung bedarf.

7. Verwendung eines Produktes, das aus R(+)-2-[4-[(Tetrahydro-2-furyl)carbonyl]-1-piperazinyl]-6,7-dimethoxy-4-chinazolinamin besteht und eine enantiomere Reinheit von wenigstens 91% aufweist, oder eines pharmazeutisch verträglichen Salzes oder Hydrates davon, das die gleiche enantiomere Reinheit aufweist, zur Herstellung eines Medikamentes zur Behandlung von gutartiger prostatischer Hyperplasie bei einem Säugerpatienten, der einer solchen Behandlung bedarf.

**8.** Verwendung eines Produktes, das aus R(+)-2-[4-[(Tetrahydro-2-furyl)carbonyl]-1-piperazinyl]-6,7-dimethoxy-4-chinazolinamin besteht und eine enantiomere Reinheit von wenigstens 91% aufweist, oder eines pharmazeutisch verträglichen Salzes oder Hydrates davon, das die gleiche enantiomere Reinheit aufweist, zur Herstellung eines Medikamentes zur Behandlung von Hyperinsulinämie bei einem Säugerpatienten, der einer solchen Behandlung bedarf.

**9.** Verwendung eines Produktes, das aus R(+)-2-[4-[(Tetrahydro-2-furyl)carbonyl]-1-piperazinyl]-6,7-dimethoxy-4-chinazolinamin besteht und eine enantiomere Reinheit von wenigstens 91% aufweist, oder eines pharmazeutisch verträglichen Salzes oder Hydrates davon, das die gleiche enantiomere Reinheit aufweist, zur Herstellung eines Medikamentes zur Behandlung von kongestivem Herzversagen bei einem Säugerpatienten, der einer solchen Behandlung bedarf.

**10.** Verwendung nach einem der Ansprüche 5-8, wobei das Produkt aus R(+)-2-[4-[(tetrahydro-2-fury)carbonyl]-1-piperazinyl]-6,7-dimethoxy-4-chinazolinaminhydrochloriddihydrat besteht, das eine enantiomere Reinheit von wenigstens 91% aufweist.

**Revendications**

**Revendications pour les Etats contractant suivants : AT, BE, CH, LI, DE, DK, FR, GB, IT, LU, NL, SE**

**1.** Produit constitué de R(+)-2-[4-[(tétrahydro-2-furanyl)carbonyl]-1-pipérazinyl]-6,7-diméthoxy-4-quinazolinamine ayant une pureté énantiomère d'au moins 91% ou l'un de ses sels ou de ses hydrates acceptable du point de vue pharmaceutique, ayant la même pureté énantiomère.

**2.** Produit constitué de chlorhydrate de R(+)-2-[4-[(tétrahydro-2-furanyl)carbonyl]-1-pipérazinyl]-6,7-diméthoxy-4-quinazolinamine dihydraté ayant une pureté énantiomère d'au moins 91%.

**3.** Composition pharmaceutique comprenant une quantité acceptable du point de vue thérapeutique du produit tel que défini par la revendication 1 ou la revendication 2, en combinaison avec un véhicule acceptable du point de vue pharmaceutique.

**4.** Produit tel que défini dans la revendication 1 ou la revendication 2, destiné à une utilisation comme agent thérapeutique.

**5.** Utilisation d'un produit tel que défini dans la revendication 1 ou la revendication 2 pour la fabrication d'un médicament pour le traitement d'états pathologiques caractérisés par des niveaux anormalement élevés d'activité adrénergique $\alpha_1$ chez un mammifère nécessitant un tel traitement.

**6.** Utilisation d'un produit tel que défini dans la revendication 1 ou la revendication 2 pour la fabrication d'un médicament pour le traitement de l'hypertension chez un mammifère nécessitant un tel traitement.

**7.** Utilisation d'un produit tel que défini dans la revendication 1 ou la revendication 2 pour la fabrication d'un médicament pour le traitement de l'hypertrophie de la prostate chez un mammifère nécessitant un tel traitement.

**8.** Utilisation d'un produit tel que défini dans la revendication 1 ou la revendication 2 pour la fabrication d'un médicament pour le traitement de l'hyperinsulémie chez un mammifère nécessitant un tel traitement.

**9.** Utilisation d'un produit tel que défini dans la revendication 1 ou la revendication 2 pour la fabrication d'un médicament pour le traitement de l'insuffisance cardiaque congestive chez un mammifère nécessitant un tel traitement.

**Revendications pour l'Etat contractant suivant : ES**

**1.** Procédé de préparation d'un produit constitué de R(+)-2-[4-[(tétrahydro-2-furanyl)carbonyl]-1-pipérazinyl]-6,7-diméthoxy-4-quinazolinamine ayant une pureté énantiomère d'au moins 91% ou l'un de ses sels ou de ses hydrates acceptable du point de vue pharmaceutique ayant la même pureté énantiomère, comprenant les étapes consistant à

a) résoudre l'acide tétrahydro-2-furoïque racémique pour obtenir l'acide R(+)-tétrahydro-2-furoïque;

b) faire réagir le produit de l'étape a) avec de la 4-amino-6,7-diméthoxy-2-pipérazinyl-quinazoline dans un solvant polaire aprotique pour produire de la R(+)-2-[4-[(tétrahydro-2-furanyl)carbonyl]-1-pipérazinyl]-6,7-diméthoxy-4-quinazolinamine ; et

c) convertir ensuite le produit de l'étape b) en un sel ou un hydrate acceptable du point de vue pharmaceutique.

2. Procédé de la revendication 1, dans lequel ladite étape a) de résolution de l'acide tétrahydro-2-furoïque racémique comprend la formation d'un mélange de sels diastéréoisomères par réaction d'acide tétrahydro-2-furoïque racémique avec de la (-)-brucine, puis la séparation du sel de (-)-brucine d'acide R(+)-tétrahydro-2-furoïque par recristallisation et, enfin, l'acidification dudit sel pour obtenir l'acide R(+)-tétrahydro-2-furoïque.

3. Procédé de la revendication 1, dans lequel l'étape a) de résolution de l'acide tétahydro-2-furoïque racémique comprend (1) la formation d'ester benzylique d'acide tétrahydro-2-furoïque racémique ; ensuite (2) la soumission de l'ester benzylique d'acide tétrahydro-2-furoïque racémique à une hydrolyse enzymatique par une enzyme qui hydrolyse de préférence l'ester benzylique d'acide S(-)-tétrahydro-2-furoïque pour former un mélange d'acide S(-)-tétrahydro-2-furoïque et d'ester benzylique de R(+)-acide tétrahydro-2-furoïque racémique ; (3) l'extraction de l'acide S(-)-tétrahydro-2-furoïque dudit mélange ; et (4) la soumission de l'ester benzylique d'acide R(+)-tétrahydro-2-furoïque à une hydrolyse acide pour obtenir l'acide R(+)-tétrahydro-2-furoïque.

4. Procédé de la revendication 1, dans lequel dans l'étape c), le produit de l'étape b) est converti en sel de chlorhydrate dihydraté.

**Revendications pour l'Etat contractant suivant : GR**

1. Procédé de préparation d'un produit constitué de R(+)-2-[4-[(tétrahydro-2-furanyl)carbonyl]-1-pipérazinyl]-6,7-diméthoxy-4-quinazolinamine ayant une pureté énantiomère d'au moins 91% ou l'un de ses sels ou de ses hydrates acceptable du point de vue pharmaceutique ayant la même pureté énantiomère, comprenant les étapes consistant à

a) résoudre l'acide tétrahydro-2-furoïque racémique pour obtenir l'acide R(+)-tétrahydro-2-furoïque ;

b) faire réagir le produit de l'étape a) avec de la 4-amino-6,7-diméthoxy-2-pipérazinyl-quinazoline dans un solvant polaire aprotique pour produire de la R(+)-2-[4-[(tétrahydro-2-furanyl)carbonyl]-1-pipérazinyl]-6,7-diméthoxy-4-quinazolinamine ; et

c) convertir ensuite le produit de l'étape b) en un sel ou un hydrate acceptable du point de vue pharmaceutique.

2. Procédé de la revendication 1, dans lequel ladite étape a) de résolution de l'acide tétrahydro-2-furoïque racémique comprend la formation d'un mélange de sels diastéréoisomères par réaction d'acide tétrahydro-2-furoïque racémique avec de la (-)-brucine, puis la séparation du sel de (-)-brucine d'acide R(+)-tétrahydro-2-furoïque par recristallisation et, enfin, l'acidification dudit sel pour obtenir l'acide R(+)-tétrahydro-2-furoïque.

3. Procédé de la revendication 1, dans lequel l'étape a) de résolution de l'acide tétahydro-2-furoïque racémique comprend (1) la formation d'ester benzylique d'acide tétrahydro-2-furoïque racémique ; ensuite (2) la soumission de l'ester benzylique d'acide tétrahydro-2-furoïque racémique à une hydrolyse enzymatique par une enzyme qui hydrolyse de préférence l'ester benzylique d'acide S(-)-tétrahydro-2-furoïque pour former un mélange d'acide S(-)-tétrahydro-2-furoïque et d'ester benzylique de R(+)-acide tétrahydro-2-furoïque racémique ; (3) l'extraction de l'acide S(-)-tétrahydro-2-furoïque dudit mélange ; et (4) la soumission de l'ester benzylique d'acide R(+)-tétrahydro-2-furoïque à une hydrolyse acide pour obtenir l'acide R(+)-tétrahydro-2-furoïque.

4. Procédé de la revendication 1, dans lequel dans l'étape c), le produit de l'étape b) est converti en sel de chlorhydrate dihydraté.

5. Utilisation d'un produit constitué de R(+)-2-[4-[(tétrahydro-2-furanyl)carbonyl]-1-pipérazinyl]-6,7-diméthoxy-4-quinazolinamine ayant une pureté énantiomère d'au moins 91% ou de l'un de ses sels ou de ses hydrates acceptable du point de vue pharmaceutique ayant la même pureté énantiomère pour la fabrication d'un médicament pour le traitement d'états pathologiques caractérisés par des niveaux anormalement élevés d'activité adrénergique $\alpha_1$ chez un mammifère nécessitant un tel traitement.

6. Utilisation d'un produit constitué de R(+)-2-[4-[(tétrahydro-2-furanyl)carbonyl]-1-pipérazinyl]-6,7-diméthoxy-4-quinazolinamine ayant une pureté énantiomère d'au moins 91% ou de l'un de ses sels ou de ses hydrates acceptable du point de vue pharmaceutique ayant la même pureté énantiomère pour la fabrication d'un médicament pour le traitement de l'hypertension chez un mammifère nécessitant un tel traitement.

7. Utilisation d'un produit constitué de R(+)-2-[4-[(tétrahydro-2-furanyl)carbonyl]-1-pipérazinyl]-6,7-diméthoxy-4-quinazolinamine ayant une pureté énantiomère d'au moins 91% ou de l'un de ses sels ou de ses hydrates acceptable du point de vue pharmaceutique ayant la même pureté énantiomère pour la fabrication d'un médicament pour le traitement de l'hypertrophie de la prostate chez un mammifère nécessitant un tel traitement.

8. Utilisation d'un produit constitué de R(+)-2-[4-[(tétrahydro-2-furanyl)carbonyl]-1-pipérazinyl]-6,7-diméthoxy-4-quinazolinamine ayant une pureté énantiomère d'au moins 91% ou de l'un de ses sels ou de ses hydrates acceptable du point de vue pharmaceutique ayant la même pureté énantiomére pour la fabrication d'un médicament pour le traitement de l'hyperinsulémie chez un mammifère nécessitant un tel traitement.

9. Utilisation d'un produit constitué de R(+)-2-[4-[(tétrahydro-2-furanyl)carbonyl]-1-pipérazinyl]-6,7-diméthoxy-4-quinazolinamine ayant une pureté énantiomère d'au moins 91% ou de l'un de ses sels ou de ses hydrates acceptable du point de vue pharmaceutique ayant la même pureté énantiomère pour la fabrication d'un médicament pour le traitement de l'insuffisance cardiaque congestive chez un mammifère nécessitant un tel traitement.

10. Utilisation d'un produit selon l'une quelconque des revendications 5 à 8, dans laquelle ledit produit est constitué de chlorhydrate de R(+)-2-[4-[(tétrahydro-2-furanyl)carbonyl]-1-pipérazinyl]-6,7-diméthoxy-4-quinazolinamine dihydraté ayant une pureté énantiomère d'au moins 91%.